(19) Europäisches Patentamt
European Patent Office
Office européen des brevets

(11) EP 2 174 930 A1

(12) # EUROPEAN PATENT APPLICATION
published in accordance with Art. 153(4) EPC

(43) Date of publication:
**14.04.2010 Bulletin 2010/15**

(21) Application number: **08778114.2**

(22) Date of filing: **11.07.2008**

(51) Int Cl.:
***C07D 211/92*** (2006.01) ***A61K 31/664*** (2006.01)
***A61P 35/00*** (2006.01) ***A61P 43/00*** (2006.01)

(86) International application number:
**PCT/JP2008/062599**

(87) International publication number:
**WO 2009/008508 (15.01.2009 Gazette 2009/03)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.07.2007 JP 2007182594**

(71) Applicants:
- **Microbial Chemistry Research Foundation Tokyo 141-0021 (JP)**
- **Nippon Kayaku Kabushiki Kaisha Chiyoda-ku Tokyo 102-8172 (JP)**

(72) Inventors:
- **AKIYAMA, Tetsuo Tokyo 141-0021 (JP)**
- **MASUDA, Toru Tokyo 141-0021 (JP)**
- **ABE, Masatoshi Tokyo 115-8588 (JP)**

(74) Representative: **Hill, Christopher Michael**
**Page White & Farrer**
**Bedford House**
**John Street**
**London, WC1N 2BF (GB)**

(54) **ANTITUMOR AGENT COMPRISING SULFOSTIN AND SULFOSTIN-RELATED COMPOUND AS THE ACTIVE INGREDIENT**

(57) The present invention aims to provide an excellent anti-tumor agent that can specifically and effectively inhibit the proliferation of tumor cells in vivo, involves a low degree of side effects, and has high safety; and a pharmaceutical composition containing the anti-tumor agent. Specifically, the present invention relates to an anti-tumor agent containing, as an active ingredient, at least one of a sulphostin-related compound represented by the following General Formula (I), a pharmacologically acceptable salt thereof and a hydrate thereof; and to a pharmaceutical composition containing the anti-tumor agent:

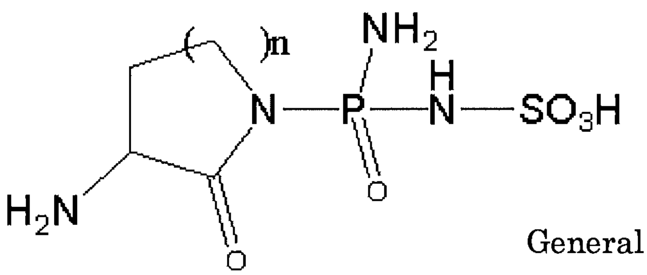

General Formula (I)

where n is an integer of 1 to 3.

EP 2 174 930 A1

**Description**

Technical Field

The present invention relates to an anti-tumor agent containing as an active ingredient sulphostin or a sulphostin-related compound, which is suitably used for the treatment of malignant tumor, especially colorectal cancer.

Background Art

Various therapies are performed on patients with cancer; among others, therapies using a chemotherapeutic agent are widely performed. Cancer cells divide more frequently than normal cells do and thus, conventionally, medicines which suppress and/or terminate the growth of cells are clinically used as an anti-tumor agent in many cases. Conventional medicines, however, cannot selectively suppress the growth of cancer cells, causing problematic side effects such as bone-marrow suppression. In view of this, development has been made for medicines that act specifically on cancer cells, and currently, such medicines have been being used clinically. But, even when these medicines are used, satisfactory clinical effects cannot be obtained. Thus, demand has arisen for a medicine that has low toxicity to normal cells and tissues and that is sufficiently expected to exhibit satisfactory therapeutic effects (see Patent Literatures 1 to 4).

The recent research reports that dipeptidyl peptidase IV (DPP-IV) inhibitor PT-100 suppresses the growth of cancer cells which have been subcutaneously transplanted into mice (see Non-Patent Literature 1). This compound, however, poses problems in the selectivity of the cell growth inhibitory activity as well as the safety of the compound itself.

Under such circumstances, there is a need to develop a novel anti-tumor agent or pharmaceutical composition that has an inhibitory activity specific to cancer cells (tumor cells), involves a low degree of side effects, and has high safety.

Patent Literature 1: Japanese Patent Application Laid-Open (JP-A) No. 08-143569
Patent Literature 2: JP-A No. 08-239379
Patent Literature 3: JP-A No. 09-249647
Patent Literature 4: JP-A No. 11-21263
Non-Patent Literature 1: Mol. Cancer Ther., Vol. 4, p. 351, 2005

Disclosure of Invention

The present invention solves the above existing problems and aims to achieve the following objects. Specifically, an object of the present invention is to provide an excellent anti-tumor agent that can specifically and effectively inhibit the proliferation of tumor cells in vivo, involves a low degree of side effects, and has high safety; and a pharmaceutical composition containing the anti-tumor agent.

The present inventors conducted extensive studies to solve the above existing problems, and as a result have obtained the following finding. Specifically, according to the finding, a sulphostin-related compound represented by General Formula (I) given below does not inhibit the proliferation of cancer cells (tumor cells) in vitro but has a potent anti-tumor effect of inhibiting the proliferation of tumor cells in vivo; and has a toxicity lower than that of PT-100, Furtulon, etc. which have conventionally known to have an anti-tumor effect. Thus, this sulphostin-related compound can be an excellent anti-tumor agent with excellent efficacy and high stability.

Sulphostin can be obtained by culturing microorganisms belonging to the Genus Streptomyses, and sulphostin-related compounds can also be obtained through chemical synthesis. These sulphostin-related compounds (which encompass sulphostin) are known to have a dipeptidyl peptidase IV inhibitory activity, and are expected to serve as, for example, a bone-marrow suppression therapeutic agent, an infection therapeutic agent, a leukocyte increasing agent, a Type 2 diabetes therapeutic agent, an immune-regulating agent, a hormone-regulating agent, an anti-HIV drug, an anti-allergic agent, an anti-inflammatory drug and an anti-rheumatoid agent (see, for example, International Publication No. WO99/25719 pamphlet; JP-A No. 2000-327689; International Publication No. WO2002/055088 pamphlet; JP-A No. 2003-183292; JP-A No. 2003-246754; JP-A No. 2004-26678; J. Antibiot., Vol. 54, p. 744, 2001; J. Nat. Prod., Vol. 67, p. 999, 2004; Bioorg. Med. Chem., Vol. 13, 785, 2005; and J. Antibiot., Vol. 58, p. 111, 2005).

However, no reports have been presented on that the sulphostin-related compounds have anti-tumor effects, and this fact is a new finding obtained by the present inventors.

The present invention is based on the finding by the present inventors. Means for solving the above existing problems are as follows.

< 1 > An anti-tumor agent containing:

as an active ingredient, at least one of a sulphostin-related compound represented by the following General

Formula (I), a pharmacologically acceptable salt thereof and a hydrate thereof:

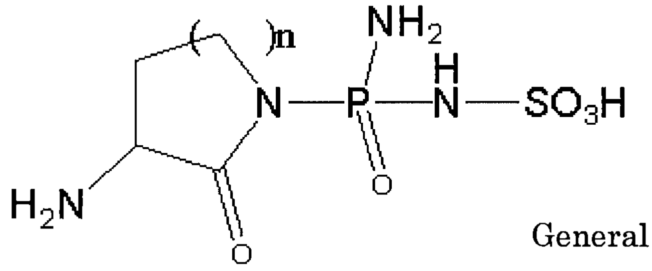

General Formula (I)

where n is an integer of 1 to 3.

< 2 > The anti-tumor agent according to < 1 > above, wherein the sulphostin-related compound is sulphostin having the following Structural Formula (II):

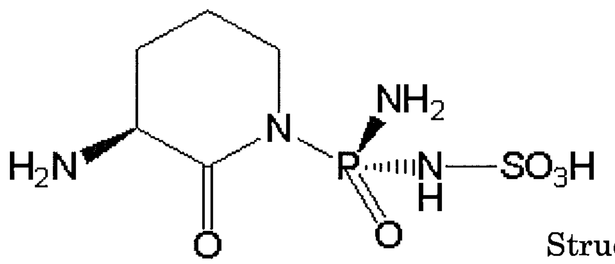

Structural Formula (II)

< 3 > A pharmaceutical composition containing:

the anti-tumor agent according to any one of < 1 > and < 2 > above.

< 4> The pharmaceutical composition according to < 3 > above, wherein the pharmaceutical composition is used for treatment of colorectal cancer.

The present invention can provide an excellent anti-tumor agent which effectively and specifically inhibits the growth of tumor cells in vivo based on a potentially new mechanism, involves a low degree of side effects, and has high safety; and a pharmaceutical composition containing the anti-tumor agent. These can solve the above existing problems.

Brief Description of Drawings

FIG. 1 is a photograph indicating the anti-tumor effect of each drug on colorectal cancer cell-transplanted mice, wherein Colon26 tumor masses taken out on Day 28 after transplantation from each mouse are shown.
FIG. 2 is a graph indicating the anti-tumor effect of each drug on colorectal cancer cell-transplanted mice, wherein the average weight, in each group, of Colon26 tumor masses taken out on Day 28 after transplantation from each mouse is shown, and each bar indicates standard deviation.
FIG. 3 is a graph indicating a change by each drug in body weight of colorectal cancer cell-transplanted mice, wherein each bar indicates standard deviation, and a decrease in body weight is observed in the PT-100 administration group.
FIG. 4 gives photographs each indicating the degree of hair loss of each colorectal cancer cell-transplanted mouse by each drug.
FIG. 5 is a graph indicating the anti-tumor effect of each drug on Day 28 after transplantation (on Day 18 after administration of the drug) when the drug is administered from Day 10 after transplantation of colorectal cancer

cells, wherein the average weight, in each group, of Colon26 tumor masses taken out from each mouse is shown, and each bar indicates standard deviation.

FIG. 6 is a graph indicating a change by each drug in body weight of mice when the drug is administered from Day 10 after transplantation of colorectal cancer cells, wherein each bar indicates standard deviation.

FIG. 7 is a graph indicating the anti-tumor effect of sulphostin on melanoma cell-transplanted mice, wherein the average weight, in each group, of B16-BL6 tumor masses taken out on Day 28 after transplantation from the mouse is shown, and each bar indicates standard deviation.

FIG. 8 is a graph indicating a change by each drug in body weight of normal mice, wherein since each dose (mg/kg) is a single dose, the daily dose is twice this dose, and each bar indicates standard deviation.

Best Mode for Carrying Out the Invention

(Anti-tumor agent)

The anti-tumor agent of the present invention contains, as an active ingredient, at least one of a sulphostin-related compound represented by the following General Formula (I), a pharmacologically acceptable salt thereof, and a hydrate thereof; and, if necessary, further contains other ingredients.

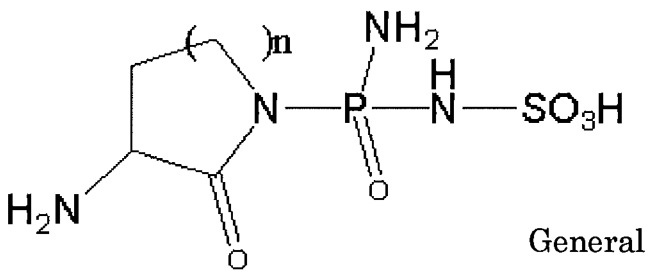

General Formula (I)

where n is an integer of 1 to 3.

< Sulphostin-related compounds >

The method for obtaining the sulphostin-related compounds represented by General Formula (I) is not particularly limited and may be appropriately selected depending on the purpose. For example, they can be produced according to any of the methods described in, for example, International Publication No. WO99/25719 pamphlet, JP-A Nos. 2000-327689, 2003-183292, 2003-246754; Akiyama T, et. al., J. Antibiot. (Tokyo), 2001, Sep; 54(9): 744-6; andAbe M, et. al., J. Nat. Prod. 2004 Jun; 67(6): 999-1,004.

Also, each of the sulphostin-related compounds represented by General Formula (I) has optically active sites at the ring-forming carbon atom (C) to which an amino group is bonded and the phosphorus atom (P) to which the other amino group is bonded and thus, there are corresponding optical isomers and racemic compounds. These optical isomers and racemic compounds are encompassed by the above sulphostin-related compounds.

Among the sulphostin-related compounds represented by General Formula (I), preferred is sulphostin having the following Structural Formula (II); i.e., General Formula (I) in which n is an integer of 2.

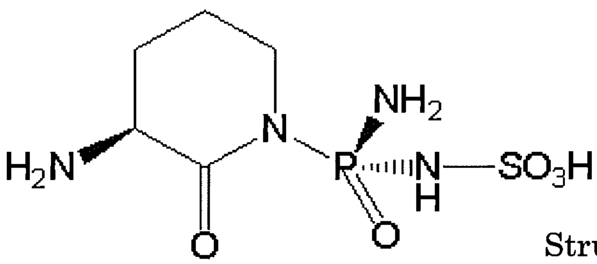

Structural Formula (II)

Notably, specific examples of the sulphostin-related compounds represented by General Formula (I) include

3(S)-amino-1-((S)-amino(sulfoamino)phosphinyl)-2-piperidone;
3(S)-amino-1-((R)-amino(sulfoamino)phosphinyl)-2-piperidone (which is a preferred sulphostin having Structural Formula (II));
3(R)-amino-1-((R)-amino(sulfoamino)phosphinyl)-2-piperidone;
3(R)-amino-1-((S)-amino(sulfoamino)phosphinyl)-2-piperidone;
3(S)-amino-1-((R)-amino(sulfoamino)phosphinyl)-2-caprolactam;
3(S)-amino-1-((S)-amino(sulfoamino)phosphinyl)-2-caprolactam;
3(R)-amino-1-((R)-amino(sulfoamino)phosphinyl)-2-caprolactam;
3(R)-amino-1-((S)-amino(sulfoamino)phosphinyl)-2-caprolactam;
3(S)-amino-1-((S)-amino(sulfoamino)phosphinyl)-2-pyrrolidone;
3(S)-amino-1-((R)-amino(sulfoamino)phosphinyl)-2-pyrrolidone;
3(R)-amino-1-((R)-amino(sulfoamino)phosphinyl)-2-pyrrolidone; and
3(R)-amino-1-((S)-amino(sulfoamino)phosphinyl)-2-pyrrolidone.

< Salt/hydrate >

In addition to the above sulphostin-related compounds, the active ingredient of the anti-tumor agent may be pharmacologically acceptable salts thereof or hydrates thereof.

The pharmacologically acceptable salt is not particularly limited and may be appropriately selected depending on the purpose. Examples thereof include salts formed from inorganic acids such as hydrochloric acid, sulfuric acid and phosphoric acid; salts formed from organic acids such as p-toluenesulfonic acid; salts formed from inorganic metals such as Na, K and Ca; and salts formed from organic amines such as methylamine, ethylamine and diethanolamine.

The above active ingredients (the sulphostin-related compounds, and pharmacologically acceptable salts thereof and/or hydrates thereof) may be used singly or in combination.

The amount of the active ingredient (the sulphostin-related compound(s), a pharmacologically acceptable salt (s) thereof and/or a hydrate(s) thereof) contained in the anti-tumor agent is not particularly limited and may be appropriately determined depending on the purpose. Further, the anti-tumor agent may be the sulphostin-related compound it self, its pharmacologically acceptable salt itself and/or its hydrate itself.

< Other ingredients >

Other ingredients are not particularly limited and may be appropriately selected depending on the purpose. Examples thereof include physiological saline which is used in, for example, dissolving and diluting the sulphostin-related compound, a pharmacologically acceptable salt thereof and/or a hydrate thereof. Further, the amount of the other ingredients contained in the anti-tumor agent is not particularly limited and may be appropriately selected depending on the purpose.

(Pharmaceutical composition)

A pharmaceutical composition of the present invention contains at least the anti-tumor agent of the present invention; and, if necessary, further contains other ingredients.

The amount of the anti-tumor agent contained in the pharmaceutical composition is not particularly limited and may be appropriately selected depending on, for example, the dosage form of the pharmaceutical composition and the intended degree of the effects. It is generally 0.01% by mass to 100% by mass, preferably 0.1% by mass to 70% by mass, as reduced to the active ingredient (the sulphostin-related compound, a pharmacologically acceptable salt thereof and/or a hydrate thereof).

< Other ingredients >

Other ingredients are not particularly limited, so long as the effects of the present invention are not impaired, and may be appropriately selected depending on the purpose. Examples thereof include pharmacologically acceptable carriers. The carrier is not particularly limited and may be appropriately selected depending on, for example, the below-described dosage form of the pharmaceutical composition. Also, the amount of the other ingredients contained in the pharmaceutical composition is not particularly limited and may be appropriately selected depending on the purpose.

< Dosage form >

The dosage form of the pharmaceutical composition is not particularly limited and may be appropriately selected depending on, for example, the below-described desired administration method. Examples thereof include oral solid preparations (e.g., tablets, coated tablets, granules, powders, powdered drugs and capsules), oral liquid preparations (e.g., internal liquid preparations, syrups and elixirs), injections (e.g., solutions, suspensions and solid preparations to be reconstituted upon use) and suppositories.

The oral solid preparations can be produced through a routine method including adding to the anti-tumor agent an excipient and other optionally used additives such as an integrating agent, a disintegrating agent, a lubricating agent, a coloring agent and a flavoring agent.

Examples of the excipient include lactose, sucrose, sodium chloride, glucose, starch, calcium carbonate, kaolin, microcrystalline cellulose and silicic acid. Examples of the integrating agent include water, ethanol, propanol, simple syrup, glucose liquid, starch liquid, gelatin liquid, carboxymethyl cellulose, hydroxypropyl cellulose, hydroxypropyl starch, methyl cellulose, ethyl cellulose, shellac, calcium phosphate and polyvinylpyrrolidone. Examples of the disintegrating agent include dry starch, sodium alginate, agar powder, sodium hydrogen carbonate, calcium carbonate, sodium lauryl sulfate, monoglyceride stearate and lactose. Examples of the lubricating agent include purified talc, stearic acid salts, borax and polyethylene glycol. Examples of the coloring agent include titanium oxide and iron oxide. Examples of the flavoring agent include sucrose, bitter orange peel, citric acid and tartaric acid.

The oral liquid preparations can be produced through a routine method including adding to the anti-tumor agent additives such as a flavoring agent, a buffer and a stabilizer.

Examples of the flavoring agent include sucrose, bitter orange peel, citric acid and tartaric acid. Examples of the buffer include sodium citrate. Examples of the stabilizing agent include tragacanth, gum arabic and gelatin.

The injections can be produced for use in subcutaneous, intramuscular and intravenous administrations through a routine method including adding to the anti-tumor agent additives such as a pH adjuster, a buffer, a stabilizer, a tonicity agent and a topical anesthetic.

Examples of the pH adjuster and buffer include sodium citrate, sodium acetate and sodium phosphate. Examples of the stabilizer include sodium pyrosulfite, EDTA, thioglycolic acid and thiolactic acid. Examples of the tonicity agent include sodium chloride and glucose. Examples of the topical anesthetic include procaine hydrochloride and lidocaine hydrochloride.

The suppositories can be produced through a routine method including adding to the anti-tumor agent known suppository carriers (e.g., polyethylene glycol, lanolin, cacao butter and fatty acid triglyceride) and an optionally used surfactant (e.g., TWEEN (registered trademark)).

[Administration]

The anti-tumor agent or the pharmaceutical composition can effectively and specifically inhibit the growth of tumor cells in vivo, involves a low degree of side effects, and have high safety. Thus, they are suitable to use as, for example, a therapeutic agent for various types of malignant tumor. The type of malignant tumor to which the anti-tumor agent or the pharmaceutical composition is applied is appropriately selected depending on the purpose. In particular, they are suitably applied to, for example, colorectal cancer and malignant melanoma; among others, suitably applied to colorectal cancer.

The anti-tumor agent or the pharmaceutical composition, therefore, can be administered to, for example, patients with various malignant tumors.

The administration method of the anti-tumor agent or the pharmaceutical composition is not particularly limited and may be appropriately selected depending on, for example, the dosage form of the pharmaceutical composition. Examples thereof include oral administration, injection and rectal administration.

The dosage amount of the anti-tumor agent or the pharmaceutical composition is not particularly limited and may be appropriately selected depending on, for example, the age and body weight of a patient to which it is to be administered and the intended degree of the effects. For example, it is preferably about 0.01 mg to about 800 mg as a daily dose for one adult, which are values reduced to the amount of the active ingredient; i.e., the sulphostin-related compound, a pharmacologically acceptable salt thereof and/or a hydrate thereof. Notably, when the continuous administration is required, the daily dose is preferably decreased.

The timing at which the anti-tumor agent or the pharmaceutical composition is administered may be appropriately selected depending on the purpose.

Notably, the animals to which the anti-tumor agent or the pharmaceutical composition is administered are not particularly limited and may be appropriately selected depending on the purpose. Examples thereof include human, mouse, rat, bovine, pig and monkey.

[Effects]

The anti-tumor agent or the pharmaceutical composition of the present invention can effectively and specifically inhibit growth of tumor cells in vivo, involves a low degree of side effects, and have high safety. Thus, they are useful as a therapeutic agent for various malignant tumors, among others, colorectal cancer.
The anti-tumor agent or the pharmaceutical composition effectively and specifically inhibits the growth of tumor cells "in vivo" (see Experimental Examples 1 to 3 described below), while does not inhibit the growth of tumor cells "in vitro" (see Experimental Example 5 described below). This indicates that the anti-tumor agent or the pharmaceutical composition can inhibit the growth of tumor cells in vivo based on a quite new mechanism different from that of a conventional anti-tumor agent. Therefore, the anti-tumor agent or the pharmaceutical composition is expected to provide a new cancer therapy excellent in therapeutic effects and safety.

Examples

The present invention will next be described by way of examples (Pharmacological Experiments and Drug Production Examples), which should not be construed as limiting the present invention thereto.
Notably, in the Examples, the "sulphostin" used was a sulphostin having Structural Formula (II). The sulphostin was obtained by culturing microorganisms belonging to the Genus Streptomyses referring to, for example, Akiyama T, et. al., J. Antibiot. (Tokyo), 2001, Sep; 54(9): 744-6; or obtained through chemical synthesis referring to, for example, Abe M, et. al., J. Nat. Prod. 2004, Jun; 67(6): 999-1,004. The "PT-100" used was synthesized referring to, for example, Mol. Cancer Ther., Vol. 4, p. 351, 2005. The "Furtulon" (generic name: Doxifluridine) used was obtained from Roche Japan K.K.
Also, in the Examples, the "suppression rate" was calculated using the following formula.

$$\text{Suppression rate (\%)} = (1 - \text{tumor weight in the administration group/tumor weight in the control group}) \times 100$$

(Experimental Example 1: Anti-tumor effect on Colon26)

Six-week-old BALB/cAnNCrj female mice were used for this test. The following six groups were provided: Group 1 in which physiological saline (solvent) was administered; Group 2 in which sulphostin was administered at a dose of 200 mg/kg; Group 3 in which sulphostin was administered at a dose of 50 mg/kg; Group 4 in which PT-100 was administered at a dose of 200 mg/kg; Group 5 in which PT-100 was administered at a dose of 50 mg/kg; and Group 6 in which Furtulon was administered at a dose of 30 mg/kg. The number of animals was set to eight in Group 1 (control group) and to six in each of Groups 2 to 6 (drug administration groups).
On Day 0, $2 \times 10^5$ Colon26 colorectal cancer cells were transplanted into the groin of the mice. Each of sulphostin and PT-100 was intraperitoneally administered to the mice at the above dose once in a day on Days 1 to 6, 8 to 13, 15 to 20 and 22 to 27. Furtulon was orally administered following a similar administration schedule. In use, sulphostin and PT-100 were each diluted with physiological saline to have an intended concentration; and Furtulon was suspended in 0.5% sodium carboxymethyl cellulose and then diluted with physiological saline to have an intended concentration. On Day 28, the tumor was taken out from each mouse and weighed, to thereby determine the effect of each drug. In addition, the presence or absence of side effects of each drug was confirmed by monitoring a change in body weight of the mouse in the course of the treatment and observing the degree of hair loss in the mouse after administration.
The results are shown in FIGs. 1 to 4.
Sulphostin was found to exhibit a potent anti-tumor effect at doses of 200 mg/kg and 50 mg/kg, at which the suppression rate was respectively 91.7% and 87.8% (significant differences: $P < 0.001$ and $P < 0.001$) with respect to the control group. In addition, at doses of 200 mg/kg and 50 mg/kg, the tumor of two out of six mice in each group was regressed. Meanwhile, PT-100 was found to exhibit an anti-tumor effect at doses of 200 mg/kg and 50 mg/kg, at which the suppression rate was respectively 62.3% and 66.7% (significant differences: $P < 0.001$ and $P < 0.001$), but the tumor was not regressed. Also, Fultulon-an existing drug-was found to exhibit an anti-tumor effect at a dose of 30 mg/kg, at which the suppression rate was 56.9% (significant difference: $P < 0.05$), but the tumor was not regressed (FIGs. 1 and 2).
Loss of body weight by continuous administration was not observed in use of sulphostin at doses of both 200 mg/kg and 50 mg/kg, but was considerably observed in use of PT-100 at a dose of 200 mg/kg (FIG. 3).
Hair loss by continuous administration was hardly observed in use of sulphostin at doses of both 200 mg/kg

and 50 mg/kg, but was extremely observed throughout the body in use of PT-100 at doses of 200 mg/kg and 50 mg/kg (FIG. 4).

These results clearly indicate that, unlike PT-100 and Furtulon, sulphostin has so potent an anti-tumor effect that the tumor is regressed and involves no such side effects as loss of body weight and hair loss.

(Experimental Example 2: Anti-tumor effect on advanced Colon26)

Six-week-old BALB/cAnNCrj female mice were used for this test. The following six groups were provided: Group 1 in which physiological saline (solvent) was administered; Group 2 in which sulphostin was administered at a dose of 50 mg/kg; Group 3 in which sulphostin was administered at a dose of 3.1 mg/kg; Group 4 in which PT-100 was administered at a dose of 50 mg/kg; Group 5 in which PT-100 was administered at a dose of 3.1 mg/kg; and Group 6 in which Furtulon was administered at a dose of 30 mg/kg. The number of animals was set to seven in Group 1 (control group) and to five in each of Groups 2 to 6 (drug administration groups).

On Day 0, $2 \times 10^5$ Colon26 colorectal cancer cells were transplanted into the groin of the mice. Administration of each drug was initiated from Day 10 when the transplanted cancer cells were thought to grow. Each of sulphostin and PT-100 was intraperitoneally administered to the mice at the above dose once in a day on Days 10 to 18 and 20 to 25. Furtulon was orally administered following a similar administration schedule. In use, sulphostin and PT-100 were each diluted with physiological saline to have an intended concentration; and Furtulon was suspended in 0.5% sodium carboxymethyl cellulose and then diluted with physiological saline to have an intended concentration. On Day 28, the tumor was taken out from each mouse and weighed, to thereby determine the effect of each drug. In addition, the presence or absence of side effects of each drug was confirmed by monitoring a change in body weight of the mouse and observing the degree of hair loss in the mouse in the course of the treatment.

The results are shown in FIGs. 5 and 6.

Sulphostin was found to exhibit a potent anti-tumor effect at doses of 50 mg/kg and 3.1 mg/kg, at which the suppression rate on tumor weight was respectively 87.3% and 66.2% (significant differences: $P < 0.001$ and $P < 0.001$) with respect to the control group. Meanwhile, PT-100 was found to exhibit an anti-tumor effect at doses of 50 mg/kg and 3.1 mg/kg, at which the suppression rate on tumor weight was respectively 70.8% and 51.5% (significant differences: $P < 0.001$ and $P < 0.001$) with respect to the control group. But, the anti-tumor effect of PT-100 was weaker than that of sulphostin. Also, Fultulon-an existing drug-was found to exhibit, at a dose of 30 mg/kg, a suppression rate of 47.0% with respect to the control group (FIG. 5).

Loss of body weight by continuous administration of sulphostin was not observed. But, in the PT-100 administration group (dose: 50 mg/kg), loss of body weight and hair loss were observed (FIG. 6).

The above discussion clearly indicates that, when administered at an advanced stage of tumor growth (from Day 10), sulphostin could regulate the host's anti-tumor immune to exhibit an anti-tumor effect.

Notably, in the control group, PT-100 administration group (dose: 3.1 mg/kg) and Furtulon administration group (dose: 30 mg/kg), presumably, increase in body weight (2 g to 4 g) corresponds to that in tumor weight (2 g to 4 g) (FIG. 6).

(Experimental Example 3: Anti-tumor effect on B16-BL6)

Five-week-old C57BL/6NCrj female mice were used for this test. The following three groups were provided: Group 1 in which physiological saline (solvent) was administered; Group 2 in which sulphostin was administered at a dose of 200 mg/kg; and Group 3 in which sulphostin was administered at a dose of 50 mg/kg. The number of animals was set to seven in Group 1 (control group) and to five in each of Groups 2 and 3 (drug administration groups).

On Day 0, $2 \times 10^5$ B16-BL6 melanoma cells were transplanted into the groin of the mice. Twenty four hours after transplantation, sulphostin was intraperitoneally administered to the mice at the above respective doses once in a day on Days 1 to 5, 7 to 12, and 14 to 19. In use, sulphostin was diluted with physiological saline to have an intended concentration. On Day 21, the tumor was taken out from each mouse and weighed, to thereby determine the effect of sulphostin.

The results are shown in FIG. 7.

Sulphostin was found to exhibit a potent anti-tumor effect at doses of 200 mg/kg and 50 mg/kg, at which the suppression rate was respectively 51.7% and 53.3% with respect to the control group (FIG. 7).

(Experimental Example 4: Toxicity test on mouse)

Four-week-old female ICR mice (Crj: CD-1) were used for this test. The following six groups were provided: Group 1 in which physiological saline (solvent) was administered; Group 2 in which sulphostin was administered at a dose of 50 mg/kg; Group 3 in which sulphostin was administered at a dose of 25 mg/kg; Group 4 in which PT-100 was administered at a dose of 50 mg/kg; Group 5 in which PT-100 was administered at a dose of 25 mg/kg; Group 6 in which

PT-100 was administered at a dose of 12.5 mg/kg; and Group 7 in which PT-100 was administered at a dose of 6.3 mg/kg. Note that each of the above doses is a dose for each administration. The number of animals was set to three in each of the groups. Each drug was intraperitoneally administered to each mouse twice in a day for 10 consecutive days, and the mouse was weighed at an appropriate time and monitored over time. On Day 19 after initiation of administration, each mouse was dissected under anesthesia and examined for remarks.

A change in body weight is shown in FIG. 8, and the weight (mg) of each organ is shown in Table 1.

Table 1

| Dose per administration (mg/kg) | | | Thymus | Heart | Lung | Liver | Spleen | Right kidney | Left kidney |
|---|---|---|---|---|---|---|---|---|---|
| Normal | | Avg. | 68.7 | 125.7 | 172.3 | 1631.7 | 102.7 | 201.3 | 196.7 |
| | | S.D. | 7.6 | 10.3 | 2.3 | 157.7 | 18.5 | 11.9 | 12.3 |
| Sulphostin | 50 | Avg. | 76 | 120 | 165.3 | 1803.7 | 141.7 | 206.7 | 202.3 |
| | | S.D. | 39.9 | 10.5 | 15 | 230.1 | 33.7 | 20.2 | 7.6 |
| Sulphostin | 25 | Avg. | 63.7 | 118.3 | 182 | 1769.3 | 127.7 | 210.3 | 204.7 |
| | | S.D. | 6 | 4.5 | 4.4 | 185 | 13.3 | 16.5 | 20 |
| PT-100 | 50 | Avg. | 37.7 | 116.3 | 229 | 1891.7 | 142.3 | 230.3 | 218 |
| | | S.D. | 21.9 | 6.4 | 91 | 378.6 | 51.4 | 30.1 | 14.4 |
| PT-100 | 25 | Avg. | 69 | 118 | 187 | 1789.3 | 150.7 | 217.7 | 211.7 |
| | | S.D. | 18.3 | 3.6 | 14.7 | 97.5 | 8.1 | 18.2 | 3.1 |
| PT-100 | 12.5 | Avg. | 80.3 | 126.3 | 195.3 | 2269 | 175 | 234 | 232.3 |
| | | S.D. | 15.3 | 7.8 | 4.2 | 241.9 | 30.5 | 18 | 24.4 |
| PT-100 | 6.3 | Avg. | 67.3 | 120.7 | 163.7 | 1570.7 | 133.7 | 203 | 200.7 |
| | | S.D. | 12.9 | 8.7 | 16.2 | 229.5 | 30.4 | 31.6 | 16.6 |

In the sulphostin administration groups (doses: 50 mg/kg and 25 mg/kg), the mice did not decrease in body weight nor did they show any abnormalities during monitoring and in remarks after dissection. In contrast, in the PT-100 administration groups (doses: 50 mg/kg and 25 mg/kg), the mice decreased in body weight to a considerable extent. In addition, as a result of monitoring, the mice of the PT-100 administration groups (doses: 50 mg/kg, 25 mg/kg and 12.5 mg/kg) involved reddening of the ears, and their tips necrotized and dropped. From the remarks after dissection, in the PT-100 administration group (dose: 50 mg/kg), the thymus shrank to 55% of that in the control group; and in the PT-100 administration group (dose: 12.5 mg/kg), the liver enlarged to 1.4 times that in the control group and the spleen 1.7 times (FIG. 8 and Table 1).

These results show that sulphostin involves no side effects even after continuous administration and thus, has stability as a drug.

(Experimental Example 5: Test on cell toxicity)

The inhibitory effect of sulphostin on the proliferation of tumor cells in vitro was investigated. The culture liquid used was a 10% FCS-containing RPMI1640 medium, and incubation was performed at 37°C in 5%$CO_2$-air. EL-4 mouse thymoma cells, Colon-26 mouse colon cancer cells and B16-BL6 mouse melanoma cells were incubated at $1 \times 10^4$ cells/well for two days, and then the number of cells was measured by the MTT method. Specifically, MTT (0.4% and 10 μL) was added to each cell culture, followed by incubating for four hours. Further, 20%sodium dodecyl sulphate (SDS) was added thereto in an amount of 100 μL, and each plate was measured for absorbance at 570 nm and 690 nm using a microplate reader. Sulphostin was added to the wells simultaneously with the initiation of the tumor cell incubation.

Table 2 shows an $IC_{50}$ value in each cell type.

Table 2

| Cell type | IC50 (μg/mL) |
|---|---|
| EL4 thymoma | >100 |
| Colon26 colon cancer | >100 |

(continued)

| Cell type | IC50 (μg/mL) |
|---|---|
| B16-BL6 melanoma | >100 |

As shown in Table 2, the $IC_{50}$ value of sulphostin on mouse tumor-derived culture cells EL4, Colon26 and B16-BL6 was 100 μg/mL or higher and thus, sulphostin was found to have no cell toxicity in vitro.

(Drug Production Example 1: Production of pharmaceutical drug for intravenous administration)

Sulphostin was dissolved in 30% (w/v) polyethylene glycol 400-containing physiological saline to prepare a 0.05% solution. The resultant solution was subjected to filter sterilization, to thereby prepare a pharmaceutical drug for intravenous administration containing 15 mg of sulphostin per vial.

(Drug Production Example 2: Production of tablet)

Sulphostin (30 parts by mass), crystalline lactose (120 parts by mass), crystalline cellulose (147 parts by mass) and magnesium stearate (3 parts by mass) were mixed with one another using a V-shaped mixer, followed by tableting, to thereby produce tablets (the mass of one tablet: 300 mg).

Industrial Applicability

The anti-tumor agent or the pharmaceutical composition of the present invention can specifically and effectively inhibit the proliferation of tumor cells in vivo, involves a low degree of side effects, and has high safety and thus, is useful as a therapeutic agent for various malignant tumors, especially colorectal cancer.

**Claims**

**1.** An anti-tumor agent comprising:

as an active ingredient, at least one of a sulphostin-related compound represented by the following General Formula (I), a pharmacologically acceptable salt thereof and a hydrate thereof:

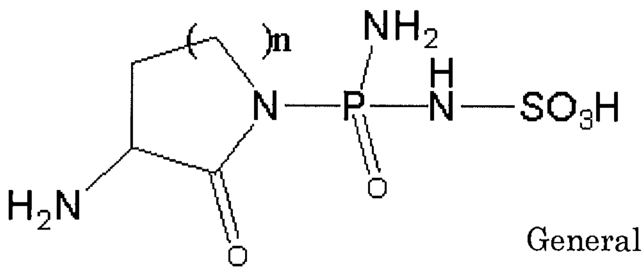

General Formula (I)

where n is an integer of 1 to 3.

**2.** The anti-tumor agent according to claim 1, wherein the sulphostin-related compound is sulphostin having the following Structural Formula (II):

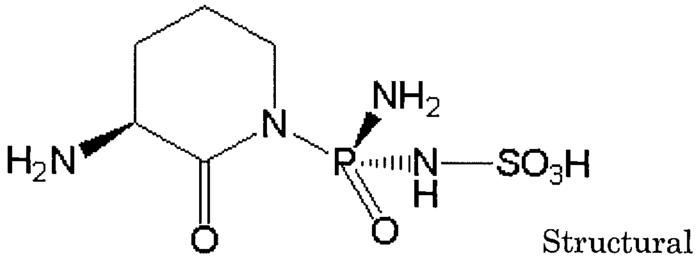

Structural Formula (II)

**3.** A pharmaceutical composition comprising:

the anti-tumor agent according to any one of claims 1 and 2.

**4.** The pharmaceutical composition according to claim 3, wherein the pharmaceutical composition is used for treatment of colorectal cancer.

FIG. 1

Control

Sulphostin
200mg/kg
50mg/kg

PT-100
200mg/kg
50mg/kg

Fultulon
30mg/kg

FIG. 2

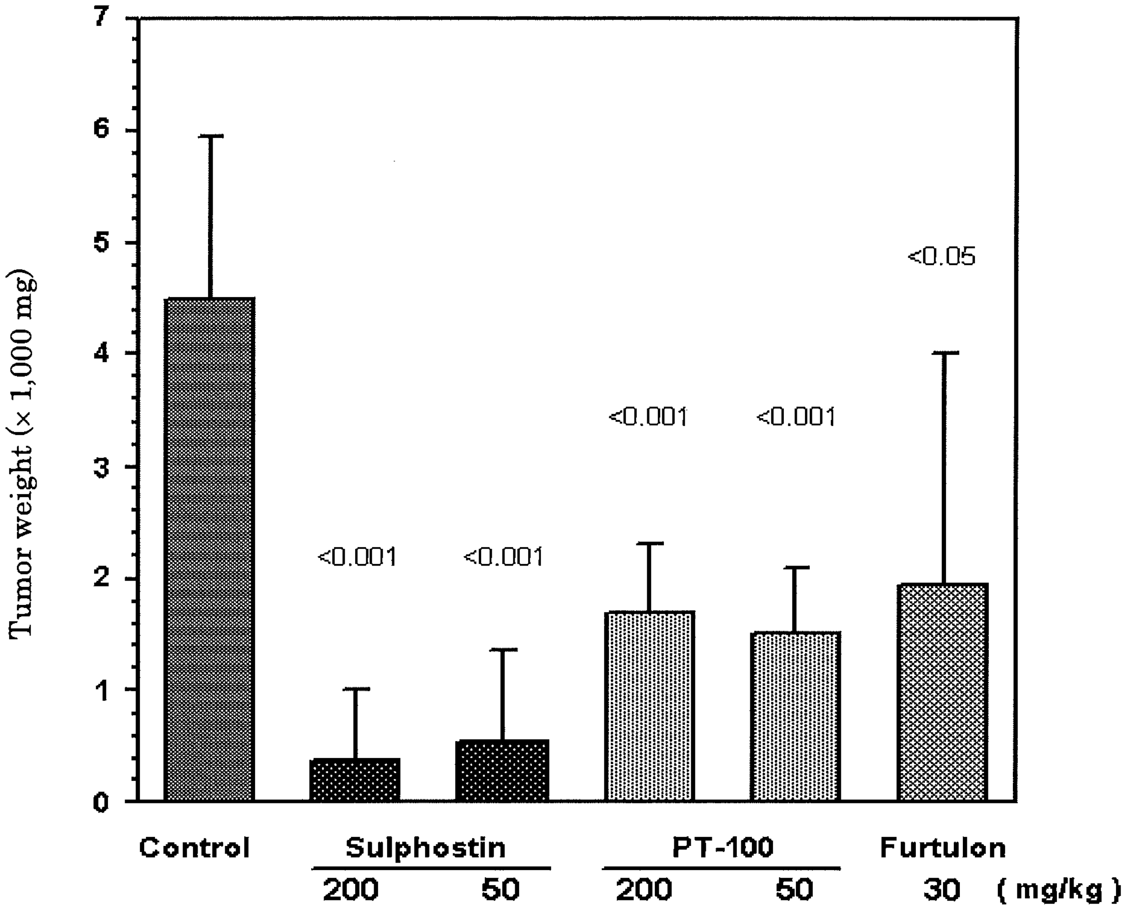
7
6
5
4
3
2
1
0
Tumor weight (× 1,000 mg)
<0.05
<0.001
<0.001
<0.001
<0.001
Control
Sulphostin
200
50
PT-100
200
50
Furtulon
30
( mg/kg )

FIG. 3

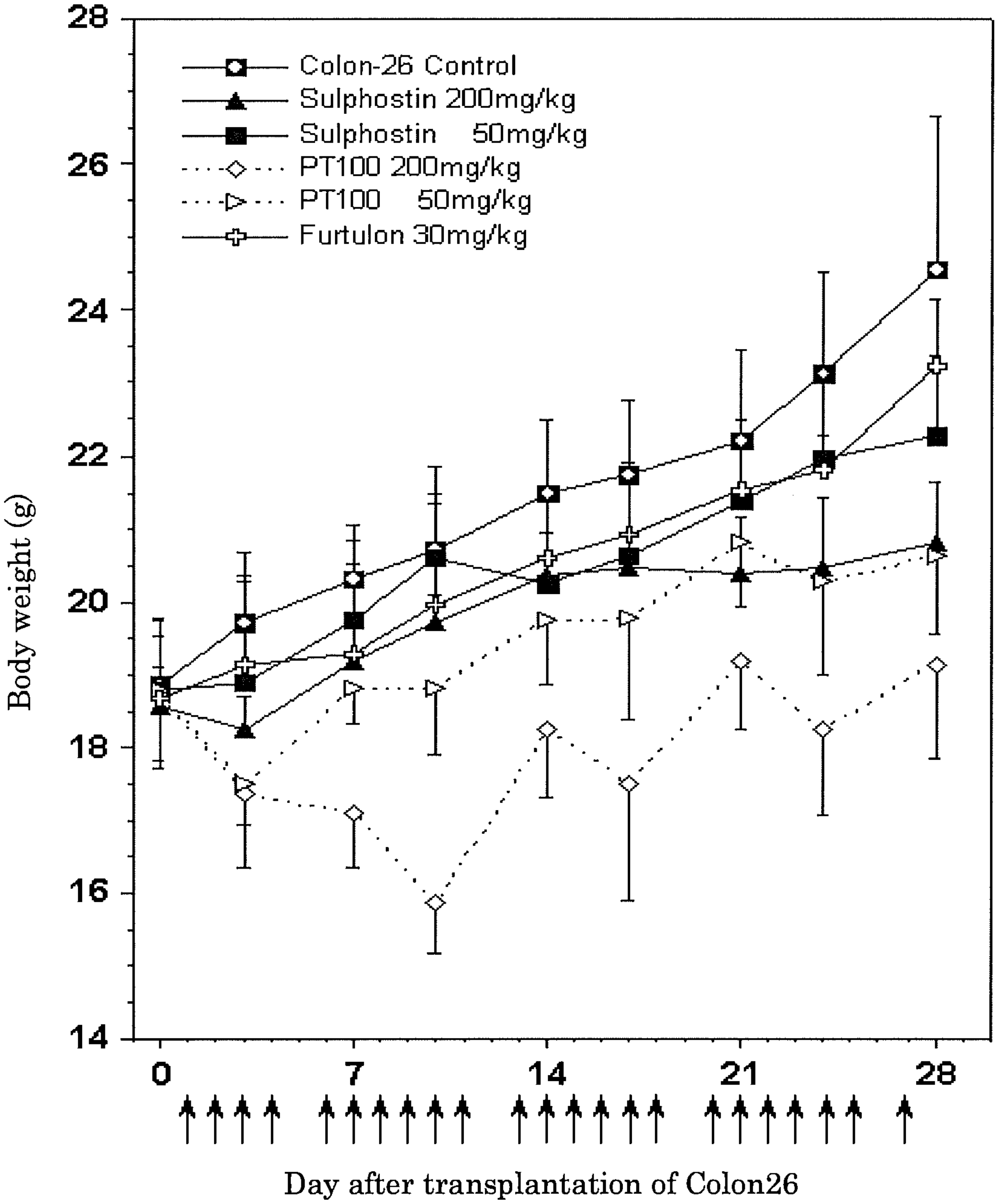
Colon-26 Control
Sulphostin 200mg/kg
Sulphostin 50mg/kg
PT100 200mg/kg
PT100 50mg/kg
Furtulon 30mg/kg
Body weight (g)
28
26
24
22
20
18
16
14
0
7
14
21
28
Day after transplantation of Colon26

FIG. 4

Control

Furtulon
30mg.kg

Sulphostin
200mg/kg

Sulphostin
50mg/kg

PT-100
200mg/kg

PT-100
50mg/kg

FIG. 5

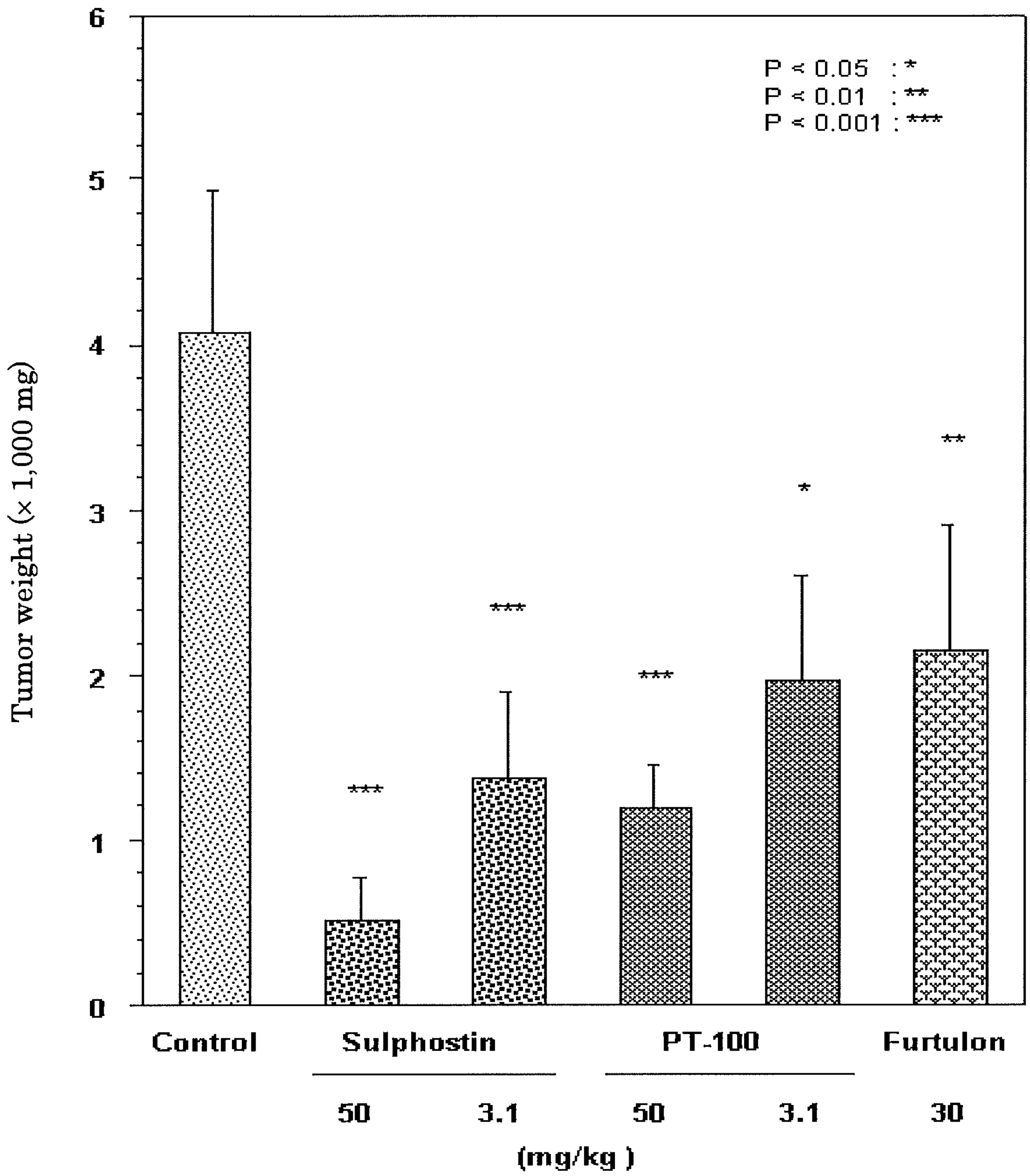
P < 0.05 : *
P < 0.01 : **
P < 0.001 : ***
Tumor weight (× 1,000 mg)
0
1
2
3
4
5
6
***
***
***
*
**
Control
Sulphostin
PT-100
Furtulon
50
3.1
50
3.1
30
(mg/kg )

FIG. 6

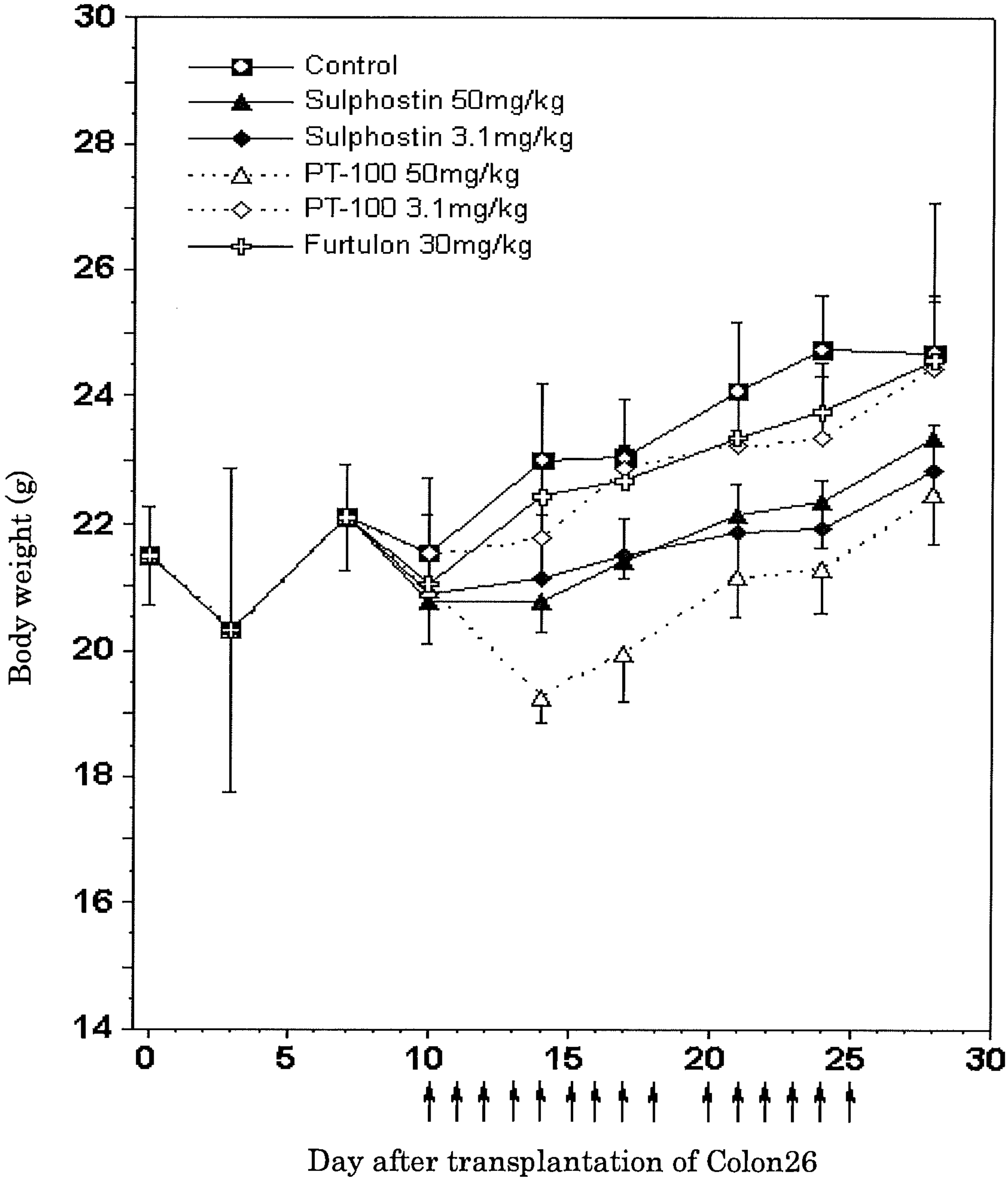

30
28
26
24
22
20
18
16
14
Body weight (g)
Control
Sulphostin 50mg/kg
Sulphostin 3.1mg/kg
PT-100 50mg/kg
PT-100 3.1mg/kg
Furtulon 30mg/kg
0
5
10
15
20
25
30
Day after transplantation of Colon26

FIG. 7

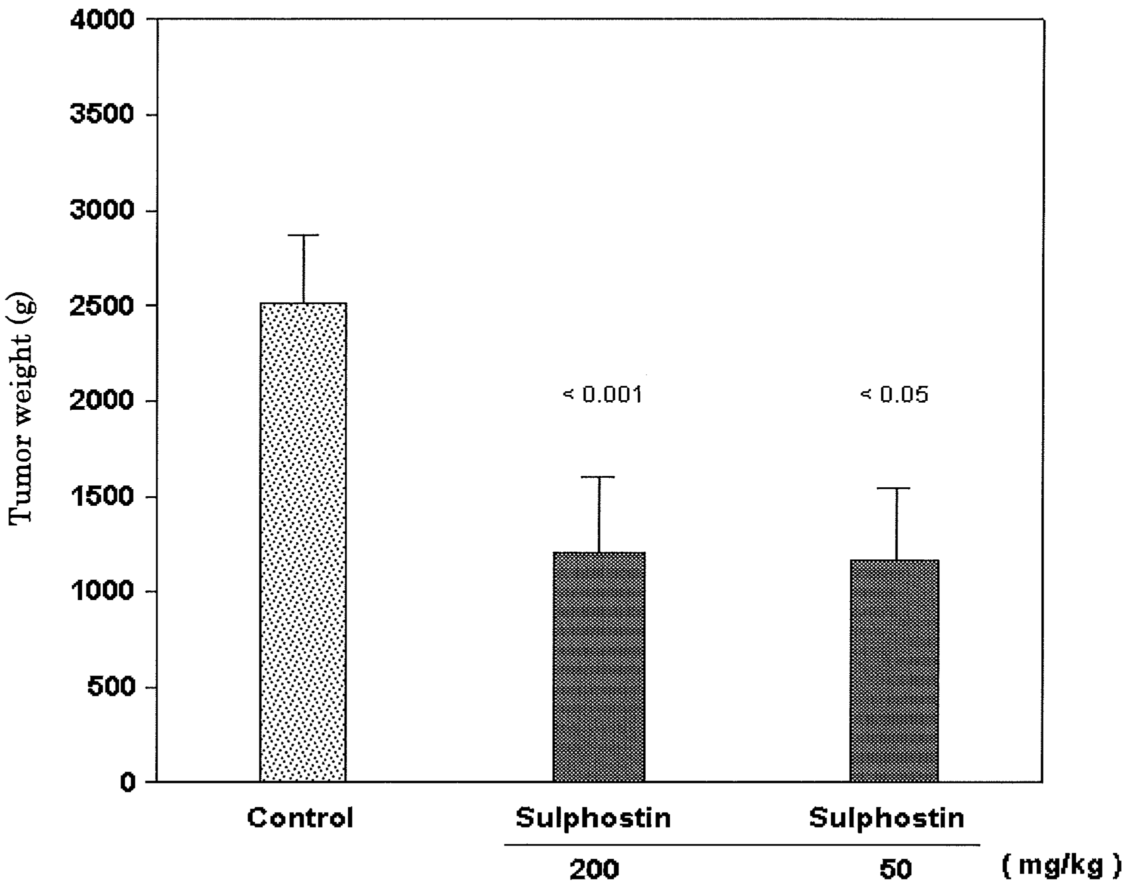
4000
3500
3000
2500
2000
1500
1000
500
0
Tumor weight (g)
< 0.001
< 0.05
Control
Sulphostin
Sulphostin
200
50
( mg/kg )

FIG. 8

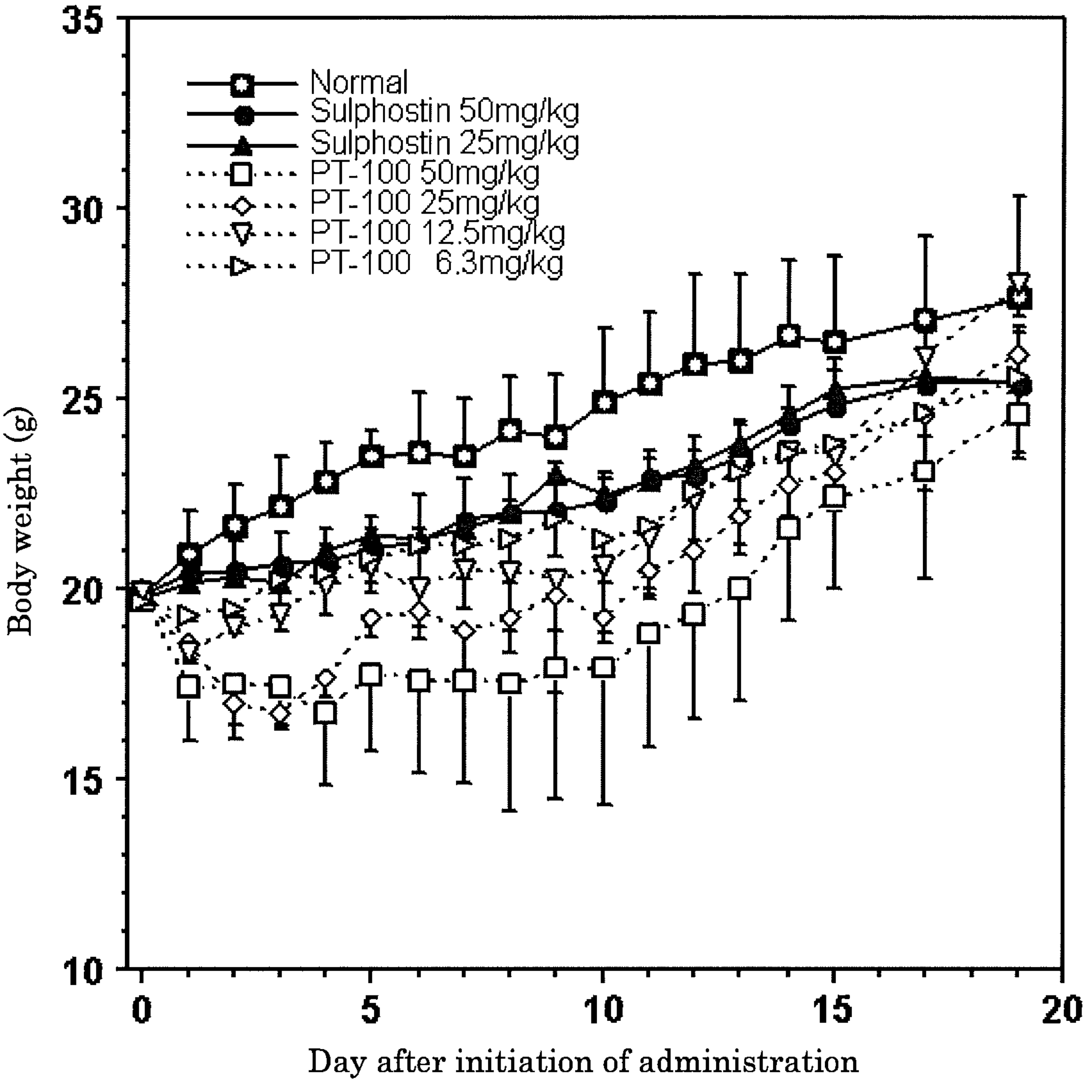
Normal
Sulphostin 50mg/kg
Sulphostin 25mg/kg
PT-100 50mg/kg
PT-100 25mg/kg
PT-100 12.5mg/kg
PT-100 6.3mg/kg
Body weight (g)
35
30
25
20
15
10
0
5
10
15
20
Day after initiation of administration

**INTERNATIONAL SEARCH REPORT**

International application No.
PCT/JP2008/062599

A. CLASSIFICATION OF SUBJECT MATTER
*C07D211/92*(2006.01)i, *A61K31/664*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D211/92, A61K31/664, A61P35/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2000-327689 A (Zaidan Hojin Biseibutsu Kagaku Kenkyu Kai), 28 November, 2000 (28.11.00), Full text & US 6579989 B1 & EP 1184388 A1 & WO 2000/069868 A1 | 1-4 |
| A | WO 2002/055088 A1 (Zaidan Hojin Biseibutsu Kagaku Kenkyu Kai), 18 July, 2002 (18.07.02), Full text & US 2004/0038942 A1 & US 2005/0256090 A1 & EP 1352655 A1 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 July, 2008 (31.07.08) | 12 August, 2008 (12.08.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/062599

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-523248 A (Guilford Pharmaceuticals), 04 August, 2005 (04.08.05), Par. No. [0006] & US 2005/0070719 A1 & EP 1465891 A2 & WO 2003/057666 A2 | 1-4 |
| A | JP 2004-43429 A (Eisai Co., Ltd.), 12 February, 2004 (12.02.04), Par. Nos. [0003], [0005] & US 2004/0082570 A1 & EP 1338595 A2 | 1-4 |
| A | ADAMS S. et al., "PH-100, a small molecule dipeptidyl peptidase inhibitor, has potent antitumor effects and augments antibody-mediated cytotoxicity via a novel immune mechanism", Cancer Research, 2004, 64, p.5471-5480, full text | 1-4 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 8143569 A
- JP 8239379 A
- JP 9249647 A
- JP 11021263 A
- WO 9925719 A
- JP 2000327689 A
- WO 2002055088 A
- JP 2003183292 A
- JP 2003246754 A
- JP 2004026678 A


### Non-patent literature cited in the description


- *Mol. Cancer Ther.,* 2005, vol. 4, 351
- *J. Antibiot.,* 2001, vol. 54, 744
- *J. Nat. Prod.,* 2004, vol. 67, 999
- *Bioorg. Med. Chem.,* 2005, vol. 13, 785
- *J. Antibiot.,* 2005, vol. 58, 111
- **Akiyama T.** *J. Antibiot. (Tokyo),* September 2001, vol. 54 (9), 744-6
- **Abe M.** *J. Nat. Prod.,* June 2004, vol. 67 (6), 999-1004